# EUROPEAN PATENT SPECIFICATION

(11) **EP 2 633 840 B1**
(45) Date of publication and mention of the grant of the patent: **24.08.2016**
(21) Application number: 13157291.9
(22) Date of filing: 28.02.2013
(51) Int. Cl.: A61F 9/00

(54) **Punctal plug with energized containment array**
Meatuspropf mit erregtem Eindämmungsarray
Bouchon méatique avec matrice de confinement sous tension

(30) Priority: 29.02.2012 US 201261604661 P
(43) Date of publication of application: 04.09.2013
(73) Proprietor: Johnson & Johnson Vision Care, Inc., Jacksonville, FL 32256 (US)
(72) Inventor: Pugh, Randall B., Jacksonville, FL 32259 (US); Martin, W. Anthony, Orange Park, FL 32073 (US)
(74) Representative: Tunstall, Christopher Stephen

(56) References cited:
- WO-A1-02/096389
- WO-A2-2004/026281
- WO-A2-2009/086112
- US-A1- 2011 301 555

## Description

### FIELD OF USE

This invention relates to a punctal plug comprising a semiconductor device as described in clam 1. Where in the following features are presented as optional this should be interpreted in such a way that protection is sought for the invention as claimed. Further disclosure relates to methods and apparatus for dispensing one or more materials, such as a medicament, from a punctal plug reservoir and dispensing a drug component in a form conducive to programmed release by electronically controlled individual dose release from an energized containment array.

### BACKGROUND

US 2011/301555 A1 relates to a porous matrix drug core for a lacrimal insert device and does not describe the feature of a semiconductor device as in claim 1.

Active agents frequently are administered to the eye for the treatment of ocular diseases and disorders. Conventional means for delivering active agents to the eye involve topical application to the surface of the eye. The eye is uniquely suited to topical administration because, when properly constituted, topically applied active agents can penetrate through the cornea and rise to therapeutic concentration levels inside the eye. Active agents for ocular diseases and disorders may be administered orally or by injection, but such administration routes are disadvantageous in that, in oral administration, the active agent may reach the eye in too low a concentration to have the desired pharmacological effect and their use is complicated by significant, systemic side effects and injections pose the risk of infection.

The majority of ocular active agents are currently delivered topically using eye drops which, though effective for some applications, are inefficient. When a drop of liquid is added to the eye, it overfills the conjunctival sac, the pocket between the eye and the lids, causing a substantial portion of the drop to be lost due to overflow of the lid margin onto the cheek. In addition, a substantial portion of the drop that remains on the ocular surface is drained into the lacrimal puncta, diluting the concentration of the drug.

To compound the problems described above, patients often do not use their eye drops as prescribed. Often, this poor compliance is due to an initial stinging or burning sensation caused by the eye drop. Certainly, instilling eye drops in one's own eye can be difficult, in part because of the normal reflex to protect the eye. Therefore, sometimes one or more drops miss the eye. Older patients may have additional problems instilling drops due to arthritis, unsteadiness, and decreased vision. Pediatric and psychiatric patient populations pose difficulties as well.

Prior topical sustained release systems include gradual release formulations, either in solution or ointment form, which are applied to the eye in the same manner as eye drops but less frequently. Such formulations are disclosed, for example, in U.S. Patent No. 3,826,258 issued to Abraham and U.S. Patent No. 4,923,699 issued to Kaufman. Due to their method of application, however, these formulations result in many of the same problems detailed above for conventional eye drops. In the case of ointment preparations, additional problems are encountered such as a blurring effect on vision and the discomfort of the sticky sensation caused by the thick ointment base.

Alternately sustained release systems have been configured to be placed into the conjunctival cul-de-sac, between the lower lid and the eye. Such units typically contain a core drug-containing reservoir surrounded by a hydrophobic copolymer membrane which controls the diffusion of the drug. Examples of such devices are disclosed in U.S. Patent No. 3,618,604 issued to Ness, U.S. Patent No. 3,626,940 issued to Zaffaroni, U.S. Patent No. 3,845,770 issued to Theeuwes et al., U.S. Patent No. 3,962,414 issued to Michaels, U.S. Patent No. 3,993,071 issued to Higuchi et al., and U.S. Patent No. 4,014,335 issued to Arnold. However, due to their positioning, the units may be uncomfortable and poor patient acceptance is again encountered.

It is known to use devices that may be inserted into one or more of an orifice of an individual's eye, such as a lacrimal punctum, to deliver active agents. One disadvantage of using such devices to deliver agents is that much of the agent may delivered in an initial, large bolus upon insertion of the device into the eye rather than a more linear delivery of the agent over time. Other methods allow for the eluding of a medicament over a period of time. However, some medicaments are most efficacious when periodically delivered in a predetermined dosed amount. Accordingly, alternative methods and devices for delivering medicaments to an ophthalmic area may be beneficial especially if discrete dosage amounts may be delivered over significant periods of time.

### SUMMARY

The present disclosure relates to devices for the discharge of medicament into the eye environment through the use of an energizing element and circuits energized by the element and contained within the body of a punctal plug.

There may be provided a semiconductor device comprising an array of containment cells, wherein each containment cell is configured to contain a medicament and each containment cell comprises a cell activation element configured to release the medicament within the containment cell upon receipt of an activation trigger.

The semiconductor device may comprise control circuitry configured to activate the containment cells at predetermined times.

The control circuitry may comprise an oscillator, a counter and a multiplexer, wherein the oscillator is configured to increment the counter, the counter is configured to output a count to the multiplexer, and the multiplexer is configured to decode the count to an address of a said containment cell.

The semiconductor device may comprise interconnection circuitry configured to route the activation trigger to a said containment cell selected by the control circuitry.

The interconnection circuitry may comprise a plurality of word lines and bit lines defining an addressable connection to the cell activation element of each containment cell.

The interconnection circuitry may comprise a unique output line for the cell activation element of each containment cell.

The control circuitry may be operable to configure the interconnection circuitry to route the activation trigger to the said selected containment cell.

Each cell activation element may comprise a fusible covering configured to contain the medicament within the cell and to release the medicament when fused.

Each fusible covering may form a hermetic seal over or around the respective containment cell.

The fusible covering may comprise a biocompatible conductor, for example a biocompatible metal, such as stainless steel, cobalt-chromium, a titanium-based alloy, Nitinol, or gold, preferably gold.

The semiconductor device may comprise fusing circuitry configured to pass a fusing current through the fusible covering of a said containment cell selected by the control circuitry, the fusing current defining the activation trigger.

The control circuitry may be configured to switch on the fusing current at the predetermined times.

The fusing circuitry may be configured to accumulate charge and to form the fusing current after a charging interval, the charging interval thereby defining the predetermined times.

The interconnection circuitry may be configured to route the fusing current from the fusing circuitry to the fusible covering of the said selected containment cell.

The semiconductor device may comprise timing circuitry configured to switch off the fusing current after a predetermined period.

Each containment cell may comprise a dissolvable layer containing the medicament within the containment cell, and wherein the cell activation element is configured to form a hermetic seal over the dissolvable layer, and to expose the dissolvable layer to the surrounding environment upon receipt of the activation trigger.

The semiconductor device may comprise engagement circuitry configured to enable the semiconductor device upon the detection of one or more predetermined conditions.

There may be provided a punctal plug configured to release discrete doses of medicament at predetermined times.

There may also be provided a punctal plug comprising control circuitry configured to activate one or more containment cells in an array to release medicament contained within the containment cells at predetermined times.

Further, there may be provided a punctal plug comprising interconnection circuitry configured to route an activation trigger to one or more selected containment cells in an array to release medicament contained within the containment cells at predetermined times.

There may be provided a yet further punctal plug comprising:
a punctal plug body with a cavity within;
an energizing device;
an engagement element to activate the connection of the energizing device to the circuits within the punctal plug;
a timing element to count for a predetermined time period;
a multiplexer to decode the timing count into a designated containment cell; and
a containment array wherein the individual elements are covered by a thin hermetic sealing film.

Any punctal plug described or claimed herein may comprise a semiconductor device as described herein.

A method of manufacturing a semiconductor device may comprise providing an array of containment cells, wherein each containment cell is configured to contain a medicament and each containment cell comprises a cell activation element configured to release the medicament within the containment cell upon receipt of an activation trigger.

The semiconductor device as described herein may be adapted for subcutaneous use.

Other embodiments are included within the scope of the following specification and claims and accompanying drawings.

### DESCRIPTION OF THE DRAWINGS

FIG. 1 illustrates a punctal plug with energized containment array.
FIG. 2 illustrates a close up depiction of medicament release features in an energized containment array.
FIG. 3 illustrates an exemplary processing flow for forming the electronics and containment array of an energized containment array.
FIG. 4 illustrates an exemplary assembly flow for assembling an energization source with electronics and a containment array into a punctal plug body.
FIGS. 5 illustrates an exemplary design for interconnections to individual medicament containers in a containment array.
FIG. 6 illustrates an exemplary design for timing circuits for medicament delivery.
FIG. 7 illustrates a block diagram of a punctal plug with energized containment array.

### DETAILED DESCRIPTION

Punctal plugs have been in use for decades now to treat conditions of dry eye. More recently they have gained attention for use as drug delivery systems for the treatment of ocular diseases and conditions. Several challenges exist with formulating a drug to release at the desired daily rate and/or dose that will give efficacy while limiting adverse events. An alternative or supplementary release strategy may involve the use of energized electronics to control and enact the delivery of individual dose amounts.

Diffusion based drug delivery systems are characterized by the drug release rate which is dependent on its diffusion through an inert water insoluble membrane barrier. There are basically two diffusion designs: reservoir devices and matrix devices. Reservoir devices are those in which a core of drug is surrounded by polymeric membrane. The nature of the membrane determines the rate of release of drug from the system. The process of diffusion is generally described by a series of equations governed by Fick's first law of diffusion. A matrix device typically consists of a drug dispersed homogenously throughout a polymer.

Reservoir and matrix drug delivery systems are considered diffusion based sustained release systems and constitute any dosage form that provides medication over an extended period of time. The goal of a sustained release system is to maintain therapeutic levels of a drug for an extended period and this is usually accomplished by attempting to obtain zero-order release from the sustained release system. Sustained release systems generally do not attain this type of release profile but try to approximate it by releasing in a slow first order manner. Over time, the drug release rate from reservoir and matrix sustained release systems will decay and become non therapeutic.

Zero-order drug release constitutes drug release from a drug delivery system at a steady sustained drug release rate, that is, the amount of drug that is released from the drug delivery system over equal time intervals does not decay and remains at the therapeutic level. This "steady sustained release drug delivery system" is referred to as a zero-order drug delivery system and has the potential to provide actual therapeutic control by its controlled release.

Another drug release profile is referred to as pulsatile drug delivery. Pulsatile drug delivery is intended to release a therapeutic amount of a therapeutic agent at regular intervals. A location for dissemination of an active agent is positioned to release the active agent into tear fluid and preferably with minimal release into the nasolacrimal duct. The pulsatile pattern is accomplished by linearly aligning water soluble encapsulated beads or other pulsatile delivery unit in a carrier, such as a tube and regulating exposure of each pulsatile delivery unit to an aqueous solution, such as tear fluid. As a first pulsatile delivery unit is exposed to the aqueous solution and dissolved, a medicament encapsulated within the pulsatile delivery unit is then released into the nasolacrimal duct. Dissolving of a first pulsatile delivery unit and consequent release of a first dose of medicament then exposes a second pulsatile delivery unit to the aqueous solution. The pattern repeats itself as the linearly aligned pulsatile delivery units are dissolved and expose a next unit to the aqueous solution.

There may be provided apparatus and methods for forming a punctal plug comprising: a punctal plug body having a first end and a second end; a surface extending between the two ends; a reservoir contained within the punctal plug body wherein the reservoir comprises an active agent-containing material and an active agent, wherein the active agent is linearly present in a pulsatile dosing bead. The punctal plug may additionally comprise a defined area, such as an opening in the punctal plug, which is more conducive to elution or other dissemination of the active agent from the punctal plug cavity to an area proximate to the punctal plug. The punctal plug may include an area conducive to dissemination of the active agent comprising an opening with a diameter which is smaller than a diameter of the cavity containing the active ingredient.

There may be provided devices, and methods for their use and manufacture, that can be used to deliver active agents into an array of cavities located at the surface of a punctal plug in a controlled manner. Through the incorporation of energization elements into the main punctal plug cavity, which are operant to energize electronic circuits also contained within the punctal plug; novel delivery of medicament doses may be realized.

It has been known to fill a cavity in a punctal plug via insertion of a rod, or other rigid or semi rigid article. The rod can include a pharmaceutical or other medicament. However, previously known administration relied upon an active agent eluding from the plug. An array of medicament containment cells may be formed in hard substrates where each containment cell is capped by a sealing means that can be made porous by the action of an applied electrical signal. This array and its associated energization, electronics and other components may be inserted into a punctal plug in a similar manner to the aforementioned filling of the cavity.

### GLOSSARY

As used herein, the term "active agent" refers to an agent capable of treating, inhibiting, or preventing a disorder or a disease. Exemplary active agents include, without limitation, pharmaceuticals and nutraceuticals. Preferred active agents are capable of treating, inhibiting, or preventing a disorder or a disease of one or more of the eye, nose and throat.

As used herein, the term "punctal plug" refers to a device of a size and shape suitable for insertion into the inferior or superior lacrimal canaliculus of the eye through, respectively, the inferior or superior lacrimal punctum.

As used herein, the term "opening" refers to an opening in the punctal plug body of a size and shape through which the active agent can pass. Preferably, only the active agent can pass through the opening. The opening may be covered with a membrane, mesh, grid 106 or it may be uncovered. The membrane, mesh, or grid may be one or more of porous, semi-porous, permeable, semi-permeable, and biodegradable.

### Energized Punctal Plug

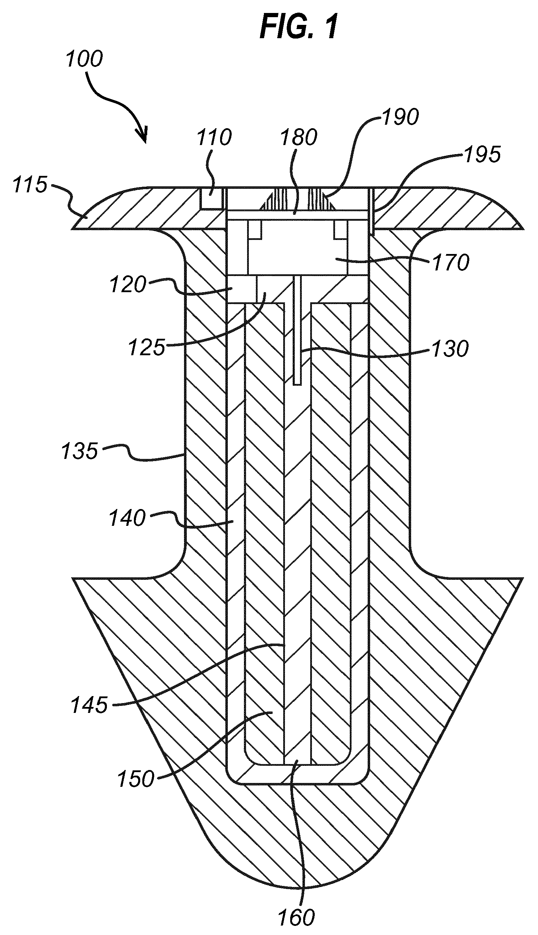

Referring to Fig. 1, item 100, an energized punctal plug is depicted. The punctal plug 100 depicted has both the elements of energization as well as components that may be energized by the energization elements. Item 135 shows an exemplary body of a punctal plug. It has a typical shape as has been described in prior disclosures. The cavity within this punctal plug body is filled with the energization elements and the medicament dispersing elements.

Item 140 demonstrates a case that may contain the energization element. In this example, the energization element is depicted as a battery and more specifically may be modeled as an alkaline cell battery. Item 150 may demonstrate the battery's cathode, which in an exemplary sense may be physically disposed upon and in contact with a metallic casing material 140. Further within the battery, element 145 may depict a separating element with an electrolyte function. Next, item 160 may then depict the anode of the battery where an electrical contacting element to the anode may be depicted as element 130. In an analogous fashion, item 120, may represent the top layer cathode contact. And to complete the energization element, item 125 may represent a top sealing cap for the element.

An alkaline battery cell is demonstrated as the energization element. Without a loss of generality there may be many other elements which may be contained within the punctal plug body which may act as energization elements including for example, other types of battery cells, fuel cells, inductively coupled energy sources, and elements of this type which may provide electrical energy to other components within the punctal plug with energized containment array device.

The energization element may be connected to a next feature, item 170, which may be an interconnect element. It may be connected in a variety of fashions to the energization element contacts 120 and 130. There may be many ways to connect the interconnect element to these contacts including solder connections, conductive epoxies, and wire-bonding for an exemplary sense. The interconnect element may optionally include a power storage element which may be charged by the energization element.

Depicted upon the interconnect device, item 180 may represent a semiconductor element which contains within its body an array of containment cells, item 190. As will be described in later description, these containment cells may be topped with a mechanism which may allow for the electrically mediated release of the material within the containment cell. There may be numerous ways to connect this semiconductor element to the interconnect element it is mounted upon, including solder ball connections that connect through vias in the semiconductor device or as is shown in Fig. 1, item 195 the interconnection may be formed through the use of a flexible connection that is run along the side of the semiconductor device and connects to interconnection positions on the top of semiconductor device.

Also shown in Fig. 1, item 115 may depict a sealing lid that is connected upon the body of the punctal plug. This sealing lid element may position and contain the energization element, interconnect array and semiconductor element in a fixed position within the punctal plug. There may be numerous manners to position and fix the sealing lid including for example the application of an epoxy sealant, or other sealant types. The lid may be formed so as to interlock with the punctal plug device with mechanical interlocks when pressed into place.

Another element depicted in Fig. 1, item 110 is an engagement device. Since the punctal plug 100 contains energization elements, the punctal plug may include means to control when the flow of current out of the energization element may occur. An engagement device may be an element that exists in an "off' position during storage and by some activation means is switched into an "on" position. Only under this condition will potential be applied to the various components in the device.

There may be numerous manners that an engagement device may be switched to its "on" position. For example, the device may be able to sense being placed into an environment that contains moisture and particularly ionic ions in water, where, for example the conductivity of such a fluid may activate an on state. Additionally a surface protecting feature may be removed from the device and thus activating the device. As the punctal plug is inserted into its human host, the device used to insert the plug into the eye's lacrimal punctum may also contain a component that effects the engagement device in some manner that then engages the device.

The engagement device may comprise a battery switch to put the timing and control circuits into a storage mode, a condition where as little current as possible is drawn from the battery. The engagement device may support visible and infrared detection through an on-chip photodetector, so one method to activate the battery switch involves incident light. Other methods include mechanical (pinching, bending), and radio frequency (RF).

### Medicament Containment Array

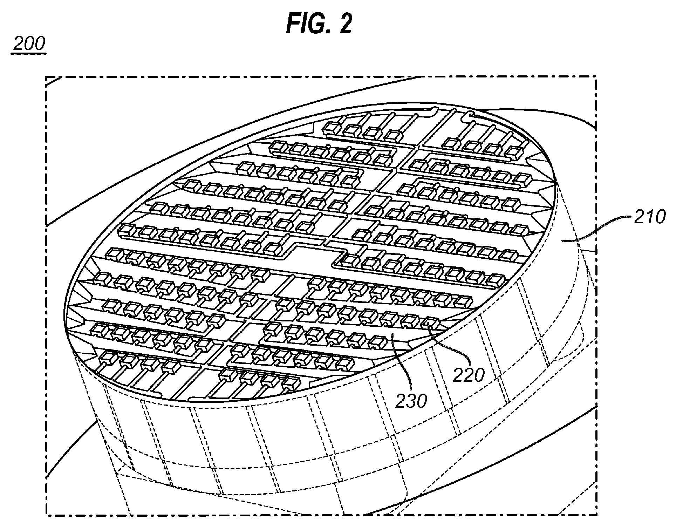

Proceeding to Fig. 2, item 200, a close up of the top surface of the semiconductor device, item 210, with the containment array is depicted. As discussed previously, the silicon piece may contain the circuitry important to controlling the containment array and ensuring that each containment cell is engaged and caused to disperse its medication at an appropriate frequency. In regions of the silicon which does not contain electronics, pores or vias may be formed into the silicon which may be filled with the medicament. On the top side of the silicon where the circuits are located, interconnect metallurgy may be used to define a matrix of regions overlying the pores or vias. Item 220, may demonstrate a metal region that lies above the pore. It will be discussed in a later section that there may be methods to form a very thin layer of metal directly above the pore. As shown by item 230, there may be metal interconnects that connect to the thin layer in such a manner that the current flow may be directed across the thin film. This current flow may cause the thin metal to melt or evaporate, in either case exposing the underlying containment cell.

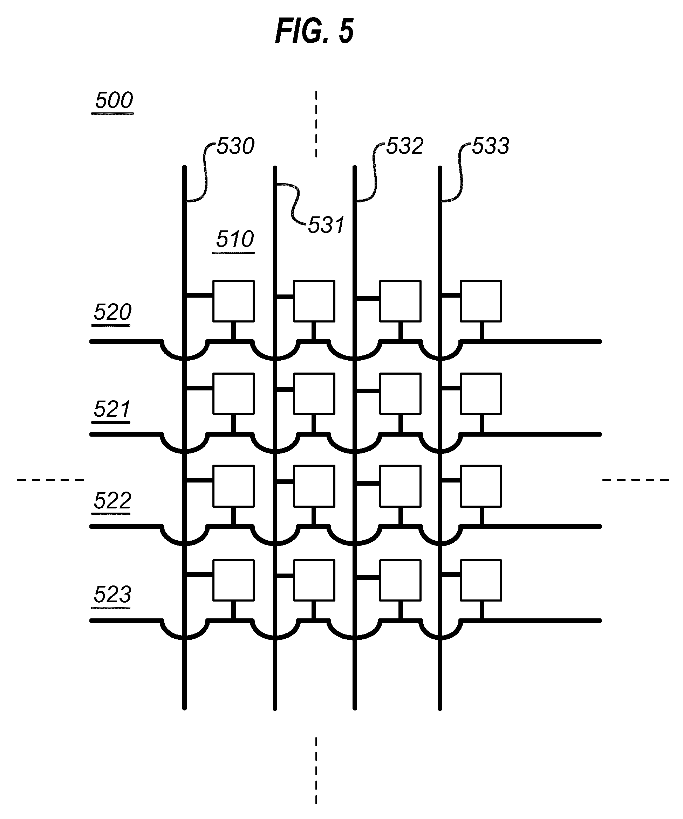

Referring to Figure 5, item 500, a depiction of routing of metal lines to allow for the connection of individual metal film layers on top of the containment array is shown. The individual cell cover layers are shown as the array of squares, one example of which is item 510. Depending on the actual size of the entire array there may be numerous additional cells that are not depicted in this figure. Also shown in the figure are a combination of 4 horizontal lines (520,521, 522 and 523), which for illustration purposes and in a similar fashion to routing for memory cells may be classified as "word lines." There are also 4 vertical lines (530, 531, 532 and 533) depicted as a subset of the "bit lines" in the array. By arranging the cells into a configuration where bit lines and word lines are capable of addressing all the containment cells an efficient scheme may be realized. For example, if it were desirable to release the medicament located under cell 510, then current may be allowed to flow through item 530, then through the cell cap 510, and then out 520.

### Timing and Control Circuits

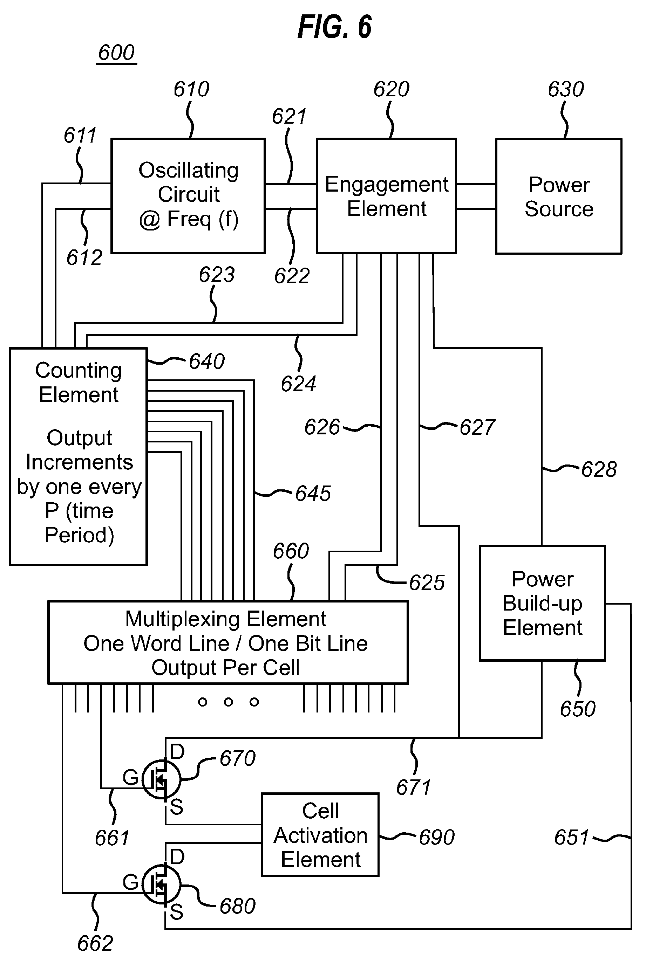

Referring now to Fig. 6, a depiction is made of an exemplary circuit to activate a particular containment cell. The circuit has a power source, 630. This power source may be an alkaline battery for example. The power may be routed from the power source to the engagement element 620. This element may be set to an "on" state when the punctal plug is placed into the eye environment. When it is set to an on state, then the power source may be routed through element 620 and out to other circuit elements. Items 621 and 622 may be the routing to an oscillating circuit element (610). Items 623 and 624 may be the routing to a counting element (640). Items 625 and 626 may be the routing to a multiplexing element (660). And, items 627 and 628 may be the routing to a Power Build-up Element (650).

Once the power is engaged in the energized punctal plug, the oscillating circuit may begin its oscillation at a particular frequency. The output of element 610 may be passed to the counting element as items 611 and 612. The counting element may have a duty cycle that counts for a certain number of cycles on the input line 612. In an exemplary sense, the combination of the frequency of oscillation and the count required before the output of the counting element increments by one may correspond to a time period of one day. Therefore, in this example, once a day the output of element 640 will be increased by one count. This count may be encoded into an eight bit number which is passed from the counting element 640 to the multiplexing element 660 through the data bus 645.

The multiplexing element 660, may receive the eight bit number and decode this number into a unique combination of a first word line, 661 and a first bit line 662. When a particular word line is activated, like line 661, it may turn on a power transistor item 670 to current flow. The bitline 662, may turn on a power transistor item 680. As was shown in Fig.5 a combination of bit line and word line may address a unique array element in the containment array. When the power transistors are engaged, power may be routed from a power build up element (650) through line 651 and then through cell 690 and out of line 671. When the power runs through the cell activation element (The thin film capping the activation cell) it may melt the thin metallic film exposing the dissolvable layers within the containment cell and then exposing the dosed medicament to the eye environment.

There may be numerous variations that are possible with this type of circuit. For example, it may be possible to use the charge up time of item 650 in concert with a resistive element to determine the timing from one cell exposure to another replacing the need for an oscillating circuit. Other variations that may be possible, include for example that the multiplexing element addresses a unique output line for every containment cell. In addition, the circuit may deliver a single cell at a particular time period. It may be apparent to one skilled in the art that various diversity may derive from an electronic controlled disbursement; including in a non-limiting sense delivering discrete drug doses from cavities at different programmed rates and programming multiple cavities to deliver doses at a particular time period in a non-limiting exemplary sense.

### Forming Electronics for Energized Containment Arrays

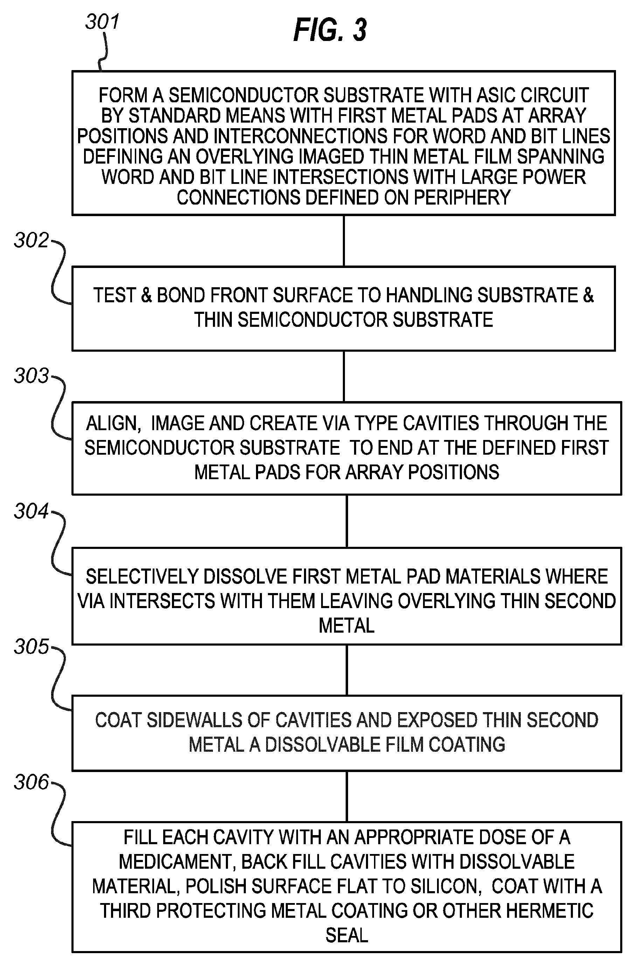

Referring to Fig. 3, a generalized process flow to form the control elements and the medicament filled containment array is depicted. In step 301, the semiconductor circuits are processed with metal layers to connect to thin metal film caps to the containment cells. In an exemplary process flow the region around a containment cell location may have a square feature built into a first metal layer of the circuit processing. This first metal layer, which may be classified as M1 may then be encapsulated with a first insulator layer. A via in this first insulator layer may define the pore through which an opened containment cell may provide medicament.

In a subsequent processing step the bit lines may be formed from a second metal layer. When this second imaged layer is etched away and then encapsulated with a second insulator layer, a second via may then be formed to allow connection to the M2 or second metal layer. A thin layer of metal may next be deposited across the two via features and then etched into discrete features where the pore is covered by the thin metal layer with then sits upon the M1 metallurgy.

Proceeding to step 302, the wafer may be tested and then a handling wafer attached or glued to the top surface of the wafer. The attached wafer may next be thinned by standard semiconductor processing steps to result in a thinned silicon layer with a flat surface. Next through silicon vias may be imaged, at step 303, and then etched to define the array of containment cells. The etching process may be processed in such a way as to etch completely through the silicon and then through insulator layers ultimately stopping on the metallization square above the particular cell.

In an next step 304, the opened through silicon vias may be subjected to a chemical etch that is capable of etching the metal layer (M1) that is exposed under each through silicon via. The etch chemistry may be chosen to be selective to the metals in M1 layer but not effective in etching the metal of the thin film layer.

Proceeding to step 305, the sidewalls and thin layer of metal present in or under the through silicon via may next be coated with a dissolvable coating. This can serve the purpose of isolating the silicon material from the medicament and also isolating the medicament from the thermal effects of the thin metal layer when it is melted or evaporated. In step 306, each of the cells is filled with an appropriate amount of medicament. Then, the cell may be capped with the dissolvable material. A subsequent polishing step may be used to planarize the backside surface down to bare silicon. Then the backside may be coated with a third metal coating deposition, or other hermetic sealing means may be employed. In this exemplary flow the electronics and the containment array may be formed.

### Assembling Punctal Plugs with Energized Containment Arrays

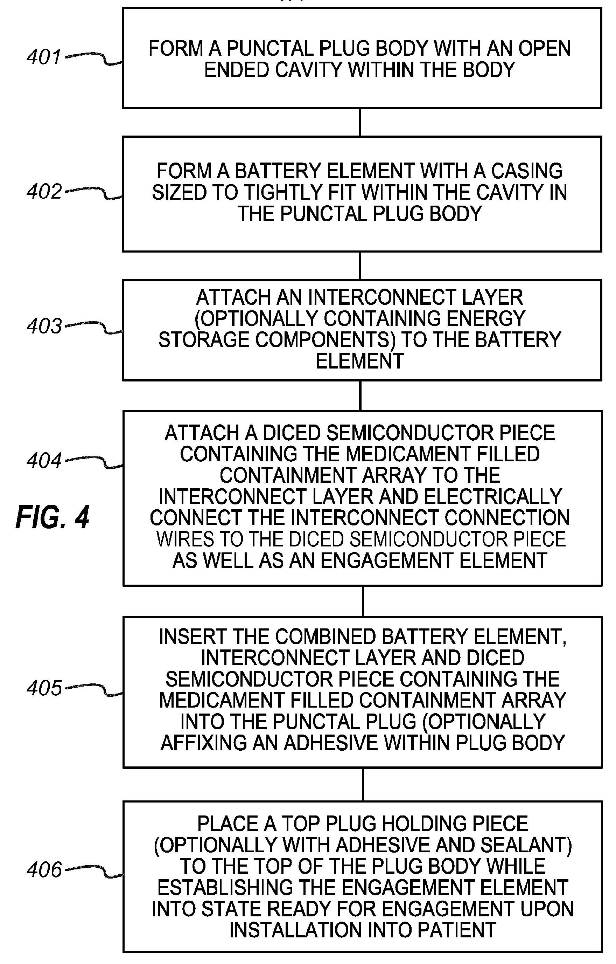

Referring now to Fig 4 an exemplary process flow for assembling the components of a Punctal Plug with an energized containment array is depicted. At step 401 the body of a punctal plug is formed. This piece may be formed by plastic molding process. Proceeding to step 402, a battery element may be produced in a manner to tightly fit into a punctal plug cavity. Next, in step 403, an interconnect layer may be attached to the battery element. There may be numerous means of making this attachment, however, from a non-limiting exemplary sense, a solder ball connection may be made between the elements.

Proceeding to step 404, the component, which may be formed by the process flow depicted in Fig. 3, is next connected to the interconnect layer. The silicon component and the interconnect layer may be electrically connected to each other through the use of flexible couplings. The resulting component which has been formed from the multiple elements may next be inserted into the cavity of a punctal plug as described in step 405. Thereafter in step 406, a top holding piece may be placed upon the punctal plug and may be useful in holding all the elements in a fixed location.

### Electrically Controlled Release of Medicament

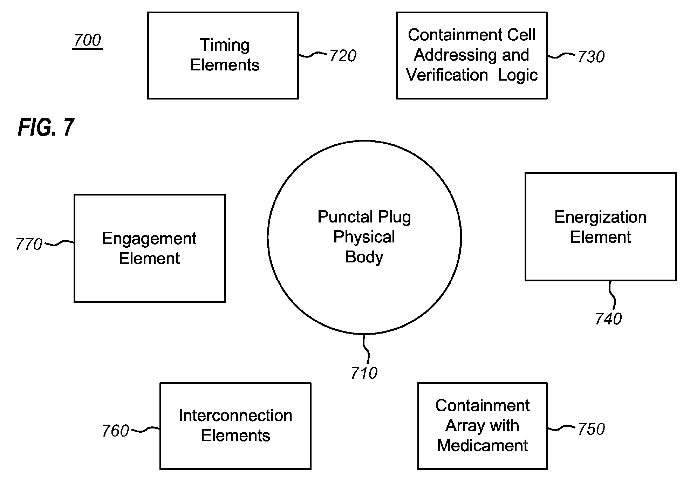

Referring now to Fig 7, item 700 the utilization of an exemplary punctal plug with energized containment array may be described in some detail. As mentioned in the previous paragraphs the formed energized punctal plug may contain all of the elements shown in Fig. 7 as items 710, 720, 730, 740, 750, 760 and 770. It may be instructive to consider how these elements may function in practice.

A punctal plug, 710, may be implanted into the lacrimal punctum of a patient's eye. In the process of placing the punctal plug in the eye the engagement element 770 may be set to an "on" state. This allows for power to be sent from an energization element 740, to all the other elements. The oscillator and counting elements, items 720, may begin to start counting. After a day, the counting element may index a position and then the multiplexer, 730, may configure a single word line and a single bit line to conduct current. This combination will define an array element within the containment array 750 and the current flow may cause the thin metal cap to melt above this first containment element. The opening in the array element may allow for tear fluid to enter the cell and dissolve the dissolvable material away. Next the medicament may be rapidly released into the eye environment in a well regulated manner. A second counter may disengage the multiplexer after a certain count has been reached so that the battery element is not discharged should a failure of the thin film to melt cause a constant current draw.

## Claims

1. A punctal plug (100) comprising a semiconductor device (180); the semiconductor device comprising:
an array of containment cells (190), wherein each containment cell is configured to contain a medicament and each containment cell comprises a cell activation element configured to release the medicament within the containment cell upon receipt of an activation trigger; and
control circuitry configured to activate the containment cells at predetermined times.

2. The punctal plug of claim 1, wherein the control circuitry comprises an oscillator, a counter and a multiplexer, wherein the oscillator is configured to increment the counter, the counter is configured to output a count to the multiplexer, and the multiplexer is configured to decode the count to an address of a said containment cell.

3. The punctal plug of claim 1 or 2, wherein the semiconductor device comprising interconnection circuitry configured to route the activation trigger to a said containment cell selected by the control circuitry.

4. The punctal plug of claim 3, wherein the interconnection circuitry comprises a plurality of word lines and bit lines defining an addressable connection to the cell activation element of each containment cell.

5. The punctal plug of claim 3, wherein the interconnection circuitry comprises a unique output line for the cell activation element of each containment cell.

6. The punctal plug of any of claims 1-5, wherein the control circuitry is operable to configure the interconnection circuitry to route the activation trigger to the said selected containment cell.

7. The punctal plug of any preceding claim, wherein each cell activation element comprises a fusible covering configured to contain the medicament within the cell and to release the medicament when fused.

8. The punctal plug of claim 7, wherein each fusible covering forms a hermetic seal over or around the respective containment cell.

9. The punctal plug of claim 7 or 8, wherein each fusible covering comprises a biocompatible conductor.

10. The punctal plug of any claims 7-9 when dependent on any of claims 1-6, comprising fusing circuitry configured to pass a fusing current through the fusible covering of a said containment cell selected by the control circuitry, the fusing current defining the activation trigger.

11. The punctal plug of claim 10, wherein the control circuitry is configured to switch on the fusing current at the predetermined times.

12. The punctal plug of claim 10, wherein the fusing circuitry is configured to accumulate charge and to form the fusing current after a charging interval, the charging interval thereby defining the predetermined times.

13. The punctal plug of any of claims 10-12 when dependent upon any of claims 4-6, wherein the interconnection circuitry is configured to route the fusing current from the fusing circuitry to the fusible covering of the said selected containment cell.

14. The punctal plug of any of claims 10-13, wherein the semiconductor device comprising timing circuitry configured to switch off the fusing current after a predetermined period.

15. The punctal plug of any preceding claim, wherein each containment cell comprises a dissolvable layer containing the medicament within the containment cell, and wherein the cell activation element is configured to form a hermetic seal over the dissolvable layer, and to expose the dissolvable layer to the surrounding environment upon receipt of the activation trigger.

16. The punctal plug of any preceding claim, wherein the semiconductor device comprising engagement circuitry configured to enable the semiconductor device upon the detection of one or more predetermined conditions.

17. The punctal plug of claim 1 comprising:
a punctal plug body with a cavity within;
an energizing device and;
an engagement element to activate a connection of the energizing device to the control circuitry within the punctal plug;
wherein the control circuitry comprising:
a timing element to count for a predetermined time period; and
a multiplexer to decode the timing count into a designated containment cell;
wherein the individual elements of the array of containment cells are covered by a thin hermetic sealing film.

## Patentansprüche

1. Punctum-Plug (100), umfassend eine Halbleitervorrichtung (180); wobei die Halbleitervorrichtung umfasst:
ein Feld von Behälterzellen (190), wobei jede Behälterzelle dafür gestaltet ist, ein Medikament zu enthalten, und jede Behälterzelle ein Zellenaktivierungselement umfasst, das dafür gestaltet ist, das Medikament in der Behälterzelle bei Empfangen eines Aktivierungsauslösers freizugeben; und
eine Steuerschaltung, die dafür gestaltet ist, die Behälterzellen zu vorbestimmten Zeitpunkten zu aktivieren.

2. Punctum-Plug gemäß Anspruch 1, wobei der Steuerschaltkreis einen Oszillator, einen Zähler und einen Multiplexer umfasst, wobei der Oszillator dafür gestaltet ist, den Zähler zu inkrementieren, der Zähler dafür gestaltet ist, eine Zählung an den Multiplexer auszugeben und der Multiplexer dafür gestaltet ist, die Zählung zu einer Adresse der Behälterzelle zu dekodieren.

3. Punctum-Plug gemäß Anspruch 1 oder 2, wobei die Halbleitervorrichtung einen Verbindungsschaltkreis umfasst, der dafür gestaltet ist, den Aktivierungsauslöser an die von dem Steuerschaltkreis ausgewählte Behälterzelle weiterzuleiten.

4. Punctum-Plug gemäß Anspruch 3, wobei der Verbindungsschaltkreis eine Vielzahl von Wortleitungen und Bitleitungen umfasst, die eine adressierbare Verbindung zu dem Zellenaktivierungselement jeder Behälterzelle definieren.

5. Punctum-Plug gemäß Anspruch 3, wobei der Verbindungsschaltkreis eine eigene Ausgangsleitung für das Zellenaktivierungselement jeder Behälterzelle umfasst.

6. Punctum-Plug gemäß einem der Ansprüche 1-5, wobei der Steuerschaltkreis funktionsfähig ist, den Verbindungsschaltkreis zu konfigurieren, den Aktivierungsauslöser an die ausgewählte Behälterzelle weiterzuleiten.

7. Punctum-Plug gemäß einem der vorstehenden Ansprüche, wobei jedes Zellenaktivierungselement eine schmelzbare Abdeckung umfasst, die dafür gestaltet ist, das Medikament in der Zelle zu halten und, wenn geschmolzen, das Medikament freizugeben.

8. Punctum-Plug gemäß Anspruch 7, wobei jede schmelzbare Abdeckung eine hermetische Abdichtung über oder um die entsprechende Behälterzelle bildet.

9. Punctum-Plug gemäß Anspruch 7 oder 8, wobei jede schmelzbare Abdeckung einen biokompatiblen Leiter umfasst.

10. Punctum-Plug gemäß einem der Ansprüche 7-9, wenn abhängig von einem der Ansprüche 1-6, umfassend einen Schmelzschaltkreis, der dafür gestaltet ist, einen Schmelzstrom durch die schmelzbare Abdeckung der von dem Steuerschaltkreis ausgewählten Behälterzelle zu leiten, wobei der Schmelzstrom den Aktivierungsauslöser definiert.

11. Punctum-Plug gemäß Anspruch 10, wobei der Steuerschaltkreis dafür gestaltet ist, den Schmelzstrom bei den vorbestimmten Zeitpunkten einzuschalten.

12. Punctum-Plug gemäß Anspruch 10, wobei der Steuerschaltkreis dafür gestaltet ist, Ladung zu akkumulieren und den Schmelzstrom nach einem Ladeintervall zu bilden, wobei das Ladeintervall dadurch die vorbestimmten Zeitpunkte definiert.

13. Punctum-Plug gemäß einem der Ansprüche 10-12, wenn abhängig von einem der Ansprüche 4-6, wobei der Verbindungsschaltkreis dafür gestaltet ist, den Schmelzstrom von dem Schmelzschaltkreis zu der schmelzbaren Abdeckung der ausgewählten Behälterzelle weiterzuleiten.

14. Punctum-Plug gemäß einem der Ansprüche 10-13, wobei die Halbleitervorrichtung einen Zeitgeberschaltkreis umfasst, der dafür gestaltet ist, den Schmelzstrom nach einer vorbestimmten Dauer abzuschalten.

15. Punctum-Plug gemäß einem der vorstehenden Ansprüche, wobei jede Behälterzelle eine auflösbare Schicht umfasst, die das Medikament in der Behälterzelle hält, und wobei das Zellenaktivierungselement dafür gestaltet ist, eine hermetische Abdichtung über der auflösbaren Schicht zu bilden und die auflösbare Schicht nach Empfangen des Aktivierungsauslösers gegenüber der umgebenden Umgebung zu exponieren.

16. Punctum-Plug gemäß einem der vorstehenden Ansprüche, wobei die Halbleitervorrichtung einen Kontaktschaltkreis umfasst, der dafür gestaltet ist, die Halbleitervorrichtung nach dem Nachweisen einer oder mehrerer vorbestimmter Bedingungen zu aktivieren.

17. Punctum-Plug gemäß Anspruch 1, umfassend:
einen Punctum-Plug-Körper mit einem Hohlraum darin;
eine Energieversorgungsvorrichtung und;
ein Kontaktelement zum Aktivieren einer Verbindung von der Energieversorgungsvorrichtung zu dem Steuerschaltkreis in dem Punctum-Plug;
wobei der Steuerschaltkreis umfasst:
ein Zeitgeberelement zum Zählen über eine vorbestimmte Zeitdauer; und
einen Multiplexer zum Dekodieren der Zeitgeberzählung zu einer ausgewiesenen Behälterzelle;
wobei die einzelnen Elemente des Felds von Behälterzellen von einem dünnen hermetischen Abdichtungsfilm bedeckt sind.

## Revendications

1. Bouchon lacrymal (100) comprenant un dispositif semi-conducteur (180) ; le dispositif semi-conducteur comprenant :
une matrice de cellules de confinement (190), chaque cellule de confinement étant configurée pour contenir un médicament et chaque cellule de confinement comprenant un élément d'activation de cellule configuré pour libérer le médicament à l'intérieur de la cellule de confinement lors de la réception d'un déclencheur d'activation ; et
un circuit de commande configuré pour activer les cellules de confinement à des temps prédéterminés.

2. Bouchon lacrymal de la revendication 1, dans lequel le circuit de commande comprend un oscillateur, un compteur et un multiplexeur, l'oscillateur étant configuré pour incrémenter le compteur, le compteur étant configuré pour transmettre un compte au multiplexeur, et le multiplexeur étant configuré pour décoder le compte à une adresse d'une desdites cellules de confinement.

3. Bouchon lacrymal de la revendication 1 ou 2, dans lequel le dispositif semi-conducteur comprend un circuit d'interconnexion configuré pour acheminer le déclencheur d'activation jusqu'à une desdites cellules de confinement sélectionnée par le circuit de commande.

4. Bouchon lacrymal de la revendication 3, dans lequel le circuit d'interconnexion comprend une pluralité de lignes de mots et de lignes de bits définissant une connexion adressable avec l'élément d'activation de cellule de chaque cellule de confinement.

5. Bouchon lacrymal de la revendication 3, dans lequel le circuit d'interconnexion comprend une ligne de sortie unique pour l'élément d'activation de cellule de chaque cellule de confinement.

6. Bouchon lacrymal de l'une quelconque des revendications 1 à 5, dans lequel le circuit de commande est utilisable pour configurer le circuit d'interconnexion afin d'acheminer le déclencheur d'activation jusqu'à ladite cellule de confinement sélectionnée.

7. Bouchon lacrymal d'une quelconque revendication précédente, dans lequel chaque élément d'activation de cellule comprend un enrobage fusible configuré pour contenir le médicament à l'intérieur de la cellule et pour libérer le médicament une fois fondu.

8. Bouchon lacrymal de la revendication 7, dans lequel chaque enrobage fusible forme un joint hermétique sur ou autour de la cellule de confinement respective.

9. Bouchon lacrymal de la revendication 7 ou 8, dans lequel chaque enrobage fusible comprend un conducteur biocompatible.

10. Bouchon lacrymal de l'une quelconque des revendications 7 à 9 lorsqu'elles sont dépendantes de l'une quelconque des revendications 1 à 6, comprenant un circuit de fusion configuré pour faire passer un courant de fusion à travers l'enrobage fusible d'une desdites cellules de confinement sélectionnée par le circuit de commande, le courant de fusion définissant le déclencheur d'activation.

11. Bouchon lacrymal de la revendication 10, dans lequel le circuit de commande est configuré pour allumer le courant de fusion aux temps prédéterminés.

12. Bouchon lacrymal de la revendication 10, dans lequel le circuit de fusion est configuré pour accumuler une charge et pour former le courant de fusion après un intervalle de charge, l'intervalle de charge définissant ainsi les temps prédéterminés.

13. Bouchon lacrymal de l'une quelconque des revendications 10 à 12 lorsqu'elles sont dépendantes de l'une quelconque des revendications 4 à 6, dans lequel le circuit d'interconnexion est configuré pour acheminer le courant de fusion depuis le circuit de fusion jusqu'à l'enrobage fusible de ladite cellule de confinement sélectionnée.

14. Bouchon lacrymal de l'une quelconque des revendications 10 à 13, dans lequel le dispositif semi-conducteur comprend un circuit de temporisation configuré pour couper le courant de fusion après une période prédéterminée.

15. Bouchon lacrymal d'une quelconque revendication précédente, dans lequel chaque cellule de confinement comprend une couche dissolvable contenant le médicament à l'intérieur de la cellule de confinement, et dans lequel l'élément d'activation de cellule est configuré pour former un joint hermétique sur la couche dissolvable, et pour exposer la couche dissolvable au milieu environnant lors de la réception du déclencheur d'activation.

16. Bouchon lacrymal d'une quelconque revendication précédente, dans lequel le dispositif semi-conducteur comprend un circuit d'engagement configuré pour activer le dispositif semi-conducteur lors de la détection d'une ou plusieurs conditions prédéterminées.

17. Bouchon lacrymal de la revendication 1 comprenant :
un corps de bouchon lacrymal avec une cavité à l'intérieur ;
un dispositif d'alimentation ; et
un élément d'engagement servant à activer une connexion du dispositif d'alimentation au circuit de commande à l'intérieur du bouchon lacrymal ;
le circuit de commande comprenant :
un élément de temporisation servant à décompter un laps de temps prédéterminé ; et
un multiplexeur servant à décoder le décompte de temps dans une cellule de confinement désignée ;
les éléments individuels de la matrice de cellules de confinement étant recouverts d'un film mince d'étanchéité hermétique.
